# FASCICULE DE BREVET EUROPEEN

(11) **EP 1 011 504 B1**
(45) Date de publication et mention de la délivrance du brevet: **08.06.2005**
(21) Numéro de dépôt: 98917288.7
(22) Date de dépôt: 01.04.1998
(51) Int. Cl.: A61B 17/70

(54) **INSTRUMENTATION D'OSTEOSYNTHESE A CONNECTEUR ENTRE TIGE VERTEBRALE ET ORGANES D'ANCRAGE**
VORRICHTUNG ZUR OSTEOSYNTHESE DIE EIN VERBINDUNGSELEMENT DER WIRBELSAÜLENSTANGE UND DER VERANKERUNGSELEMENTE AUFWEIST
APPARATUS FOR OSTEOSYNTHESIS COMPRISING A CONNECTOR OF THE SPINAL PIN AND THE ANCHORING ELEMENTS

(30) Priorité: 01.04.1997 FR 9703958
(43) Date de publication de la demande: 28.06.2000
(73) Titulaire: Chopin, Daniel, 62155 Merlimont (FR); ROUSSOULY, Pierre, 69450 Saint Cyr au Mont d'Or (FR); Grosse, Arsène, 67000 Strasbourg (FR); TAGLANG, Gilbert, 67370 Griesheim sur Souffel (FR)
(72) Inventeur: CHOPIN, Daniel, F-62155 Merlimont (FR); ROUSSOULY, Pierre, F-69450 Saint Cyr au Mont d'Or (FR); GROSSE, Arsène, F-67000 Strasbourg (FR); TAGLANG, Gilbert, F-67370 Grieshein-Souffel (FR); MOULIN, Jean, F-69006 Lyon (FR); SAURAT, Jean, F-49240 Avrille (FR)
(74) Mandataire: Jacobson, Claude
(86) Numéro de dépôt international: PCT/FR1998/000662
(87) Numéro de publication internationale: WO 1998/043551

(56) Documents cités:
- FR-A- 2 615 095
- FR-A- 2 693 365
- FR-A- 2 720 923
- FR-A- 2 736 258
- US-A- 5 498 264

## Description

La présente invention a pour objet une instrumentation d'ostéosynthèse rachidienne, du type comprenant au moins une tige vertébrale longitudinale et de préférence deux, des organes d'ancrage osseux ayant un axe longitudinal et échelonnés le long de la tige, un connecteur de liaison entre la tige et chaque organe d'ancrage, et des moyens de pression pour bloquer ensemble la tige, les organes d'ancrage et les connecteurs.

On connaît des instrumentations rachidiennes de ce genre, par exemple par la demande EP-A-0.553.424, le brevet US-A-4.648.388, US-A-5 498 264 le brevet EP-A-0.384.001 et le brevet FR-A-92 07 504 (n° de publication 2.692.471). L'organe d'ancrage peut être une vis ou un crochet pédiculaire et les moyens de pression peuvent être des écrous venant se visser sur une extrémité filetée de l'organe d'ancrage. Le connecteur ou bride de liaison peut être en deux pièces ou monobloc en formant une pince qui entoure la tige vertébrale.

Lorsqu'il est monobloc, le chirurgien doit nécessairement introduire la tige dans le connecteur suivant une direction parallèle à l'axe longitudinal de la tige, ce qui peut constituer une sujétion gênante pour le chirurgien.

Ces dispositifs sont de plus relativement encombrants en direction transversale, en raison notamment de l'écartement entre l'organe d'ancrage et la tige vertébrale.

L'invention a pour but de proposer une instrumentation moins encombrante et qui soit d'une mise en oeuvre plus aisée pour le chirurgien, tout en offrant une excellente tenue mécanique dans le temps et donc une bonne fiabilité.

Grâce à cette disposition du connecteur, la tige vient se bloquer directement sur la surface inclinée d'appui prévue sur l'organe d'ancrage lorsque les moyens de pression, par exemple un écrou, sont serrés sur le connecteur, qui applique fermement la tige sur la surface d'appui de l'organe d'ancrage. De ce fait, l'encombrement du dispositif dans la direction transversale à la tige est considérablement diminué par rapport aux instrumentations antérieures connues.

En outre, l'ouverture latérale d'introduction de la tige dans le connecteur jusqu'à son logement facilite la tâche du chirurgien car cette introduction latérale est nettement plus aisée qu'une introduction dans la direction longitudinale du logement.

Suivant d'autres caractéristiques de l'invention :
- La surface d'appui inclinée est évasée, par exemple conique, ou encore constituée par un plat.
- L'organe d'ancrage est constitué en deux parties, dont l'une porte la surface d'appui de la tige et est montée de manière amovible sur l'autre. Ainsi il peut s'agir d'une bague à surface extérieure conique, montée coulissante autour de l'organe d'ancrage et pouvant venir en butée contre un épaulement annulaire transversal dudit organe d'ancrage.
- Suivant un mode de réalisation avantageux de l'instrumentation, le logement du connecteur se prolonge, du côté opposé à l'ouverture d'introduction de la tige, par un dégagement latéral central délimitant deux griffes de part et d'autre dudit dégagement, les arêtes des parois de ces griffes et la surface d'appui de l'organe d'ancrage offrant trois points de contact à la tige après serrage des moyens de pression et déformation de la tige sous l'action de ces derniers.

Ces trois points de contact constituent un appui particulièrement solide pour la tige après blocage de l'organe de compression sur le connecteur.

D'autres particularités et avantages de l'invention apparaîtront au cours de la description qui va suivre, faite en référence aux dessins annexés qui en illustrent plusieurs formes de réalisations à titre d'exemples non limitatifs.

La figure 1 est une vue mi-coupe transversale mi-élévation partielle à échelle agrandie d'une première forme de réalisation de l'instrumentation d'ostéosynthèse rachidienne conforme à l'invention.

La figure 2 est une vue de dessus en plan à échelle réduite, avec arrachements, de l'instrumentation de la figure 1.

La figure 3 est une vue en perspective à échelle agrandie du connecteur de l'instrumentation des figures 1 et 2.

La figure 4 est une vue en coupe transversale et élévation partielle de l'instrumentation des figures 1 et 2 sans l'organe de compression du connecteur sur la tige.

La figure 5 est une vue sensiblement à l'échelle de l'instrumentation des figures 1 à 4 ancrée sur une vertèbre.

La figure 6 est une vue similaire à la figure 2 d'un second mode de réalisation de l'invention.

Les figures 7, 8 et 9 sont des vues analogues à la figure 1 d'un troisième, d'un quatrième et d'un cinquième modes de réalisation de l'invention.

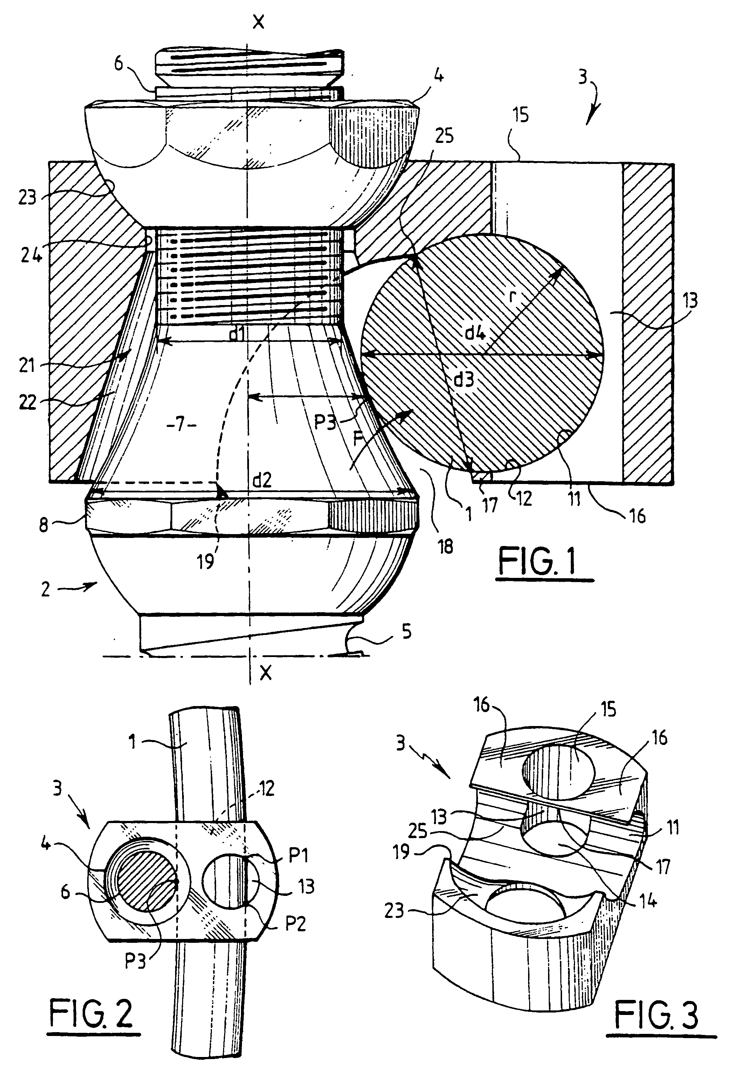

Le dispositif représenté aux figures 1 à 4 illustre partiellement une instrumentation d'ostéosynthèse rachidienne destinée à l'étaiement du rachis pour corriger des déformations tridimensionnelles de celui-ci, telles que des scolioses.

Cette instrumentation comprend au moins une tige vertébrale longitudinale 1 et de préférence deux, s'étendant le long d'un segment vertébral de deux ou plusieurs vertèbres, ainsi que des organes d'ancrage vertébral 2 ayant un axe longitudinal XX et échelonnés le long de chaque tige 1. L'instrumentation comprend également des brides ou connecteurs 3 de liaison entre la tige 1 et chaque organe d'ancrage 2, et enfin des moyens de pression, constitués dans l'exemple de la figure 1 par un écrou 4 à siège sphérique, pour bloquer ensemble la tige 1, l'organe d'ancrage 2 et le connecteur 3.

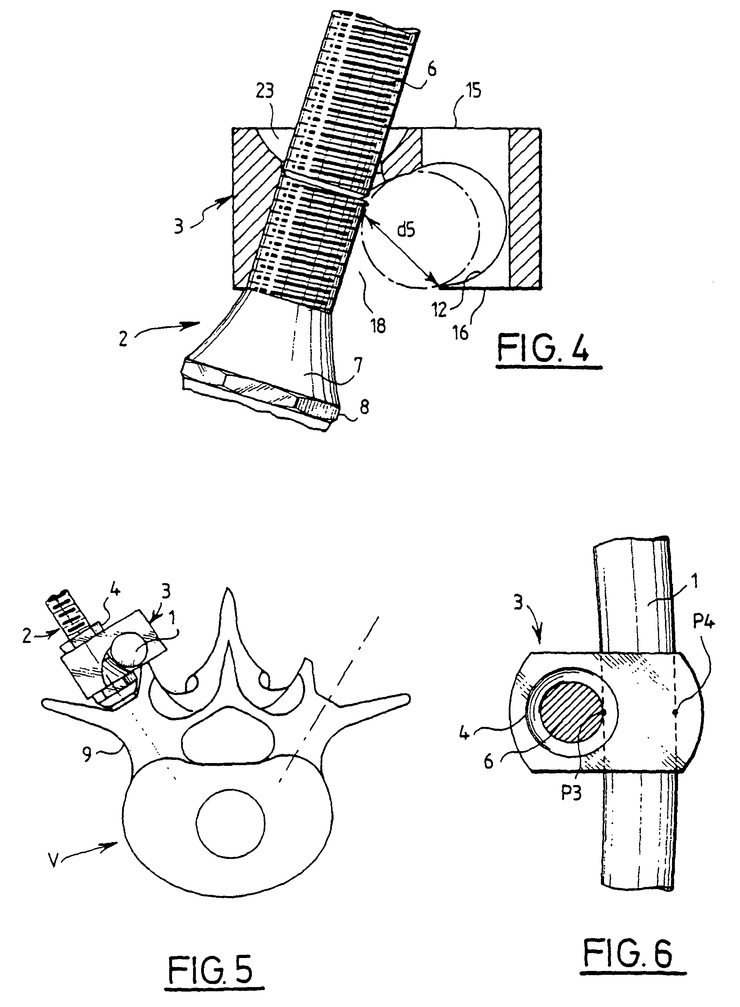

L'organe d'ancrage 2 peut être une vis pédiculaire comme partiellement représenté, ou un crochet pédiculaire, connu en soi, adapté pour être vissé ou ancré sur une vertèbre V (figure 5). La vis 2 est du type à double filetage et comporte donc une tige filetée 5 d'ancrage osseux et une partie filetée 6 reliée à la tige filetée 5 par une surface 7 de révolution autour de l'axe XX et par une forme 8 de vissage de la tige 5 dans un pédicule.

Un logement 11 destiné à la tige 1 traverse de part en part le connecteur 3 dans une direction perpendiculaire à l'axe XX lorsque l'organe d'ancrage 2 est mis en place dans le connecteur 3. Le logement 11 est délimité par deux parois cylindriques 12 ayant le même rayon de courbure que le rayon r de la tige cylindrique 1, et qui sont agencées de chaque côté d'un dégagement latéral central 13, par exemple cylindrique, agencé du côté opposé à celui prévu pour l'organe d'ancrage 2. Le dégagement 13 prolonge latéralement le logement 11 et peut comme représenté, traverser le connecteur 3 de part en part en débouchant extérieurement par des trous 14, 15. Ce dégagement 13 s'étend dans une direction perpendiculaire à celle du logement 11 et délimite ainsi, par son ouverture 15, deux griffes 16 de préhension de la tige 1 qu'elles entourent partiellement. Les griffes 16 peuvent être réunies à leurs extrémités par un raccord central 17 (figure 3), ou bien en variante ce raccord 17 peut être complètement supprimé.

Enfin le logement 11 se prolonge, du côté opposé au dégagement 13, par une ouverture latérale 18 délimitée par les extrémités des griffes 16 et par un bord intérieur opposé 19 du connecteur 3. Cette ouverture latérale a une largeur suffisante pour permettre l'introduction de la tige vertébrale 1 transversalement ou radialement à sa direction longitudinale, jusqu'à son logement 11 (symbolisée par la flèche F sur la figure 1).

Dans le connecteur 3 est également ménagé un trou traversant 21 qui s'étend coaxialement à l'axe longitudinal XX de l'organe d'ancrage 2 lui-même perpendiculaire à la tige 1. Le trou 21 comporte une première partie 22 évasée vers la tige filetée 5, dimensionnée pour pouvoir envelopper avec jeu la surface inclinée 7, et une seconde partie 23, délimitée dans l'exemple représenté par une portée sphérique raccordée à la première partie 22 par un court secteur cylindrique 24. La portion sphérique 23 constitue une portée d'appui pour la surface sphérique correspondante de l'écrou 4 lorsque celui-ci est vissé sur la partie filetée 6, l'organe d'ancrage 2 ayant été préalablement introduit dans le connecteur 3 de manière que sa partie filetée 6 traverse la seconde partie 23 du trou 21. Le trou évasé 22 se prolonge donc par l'ouverture cylindrique 24, dont le diamètre est supérieur à celui de la partie filetée 6, afin d'autoriser un débattement angulaire élevé de l'organe d'ancrage 2 par rapport au connecteur 3, comme cela sera détaillé ci-après.

La surface 7 est inclinée sur son axe de révolution XX de telle sorte que son plus petit diamètre d1 se trouve à la base de la partie filetée 6 et que son plus grand diamètre d2 se situe au niveau de la butée 8, le diamètre de cette surface 7, de révolution évasée dans l'exemple illustré à la figure 1, allant donc en croissant de la partie filetée 6 vers la butée 8.

La distance d3 entre les bords opposés de l'ouverture d'introduction 18 de la tige 1 est délimitée par une arête 25 sur la paroi intérieure de l'ouverture 18 proche de l'écrou 4, et par le bord 17 des extrémités libres des griffes 16, est sensiblement inférieure au diamètre d4 de la tige 1. Cet agencement crée un "point dur" qui doit être franchi par la tige 1, en forçant légèrement sur celle-ci après l'avoir introduite latéralement dans l'ouverture 18. Ceci permet d'insérer la tige 1 dans son logement 11, les extrémités des griffes 16 s'écartant légèrement par effet de pince élastique. Ces extrémités des griffes 16 se referment ensuite sur la tige 1, qui est ainsi maintenue par clipsage dans son logement, empêchant que cette tige ne s'échappe du connecteur 3 avant introduction de l'organe d'ancrage 2 dans ce dernier.

De plus, le logement 11, la largeur de son ouverture latérale 18 et la tige 1 sont dimensionnés de telle sorte qu'avant blocage du connecteur 3 sur la tige et sur l'organe d'ancrage 2 (situation représentée à la figure 4), l'intervalle maximum d5 entre l'organe d'ancrage 2 et le bord 17 de l'ouverture 18 formant les extrémités des griffes 16 reste toujours inférieur au diamètre d4 de la tige 1.

Cet agencement présente l'avantage de s'opposer à tout échappement de la tige 1 hors du connecteur 3 même après déclipsage au-delà de l'arête 25, dès lors que la partie filetée 6 de l'organe d'ancrage 2 traverse le connecteur 3, et quelle que soit sa position angulaire dans celui-ci. Ce débattement angulaire de l'organe d'ancrage 2 peut atteindre environ 30 degrés.

L'assemblage de l'instrumentation qui vient d'être décrite s'effectue de la manière suivante :
Le chirurgien met en place dans la vertèbre V l'organe d'ancrage 2 (figure 5); puis il introduit dans le connecteur 3 le tronçon de tige vertébrale 1 par l'ouverture latérale 18 jusqu'au logement 11, dans lequel il est retenu par l'effet de pince, en raison du point dur 25 et de la relative flexibilité des griffes 16. Puis le chirurgien enfile le connecteur 3 par ses ouvertures 22, 24, 23 sur l'organe d'ancrage 2 jusqu'à ce que la tige 1 vienne en appui sur la surface inclinée 7.
Enfin le chirurgien visse l'écrou 4 sur la partie filetée 6 jusqu'à ce que cet écrou vienne au contact du siège sphérique 23, et bloque l'ensemble de façon que le serrage de l'écrou 4 maintienne solidement la tige 1 appliquée sur la surface inclinée 7.

Le dispositif est alors dans l'état illustré aux figures 1 et 2. En fin de compression de la tige 1 et du connecteur 3 par l'écrou 4, la tige 1 subit une légère déformation, visible à la figure 2, qui a pour conséquence que sa surface s'appuie par deux points P1, P2 sur les arêtes de jonction du logement 11 et du dégagement 13. Avec le point d'appui P3 sur la surface conique 7, la tige 1 est donc bloquée par trois points d'appui P1, P2, P3.

Il convient de noter que c'est l'aménagement du dégagement latéral central 13 qui permet la création des deux points d'appui P1, P2,les trois appuis précités garantissant un excellent blocage de la tige 1 dans la position choisie, y compris dans le cas où cette dernière est cintrée.

Dans le second mode de réalisation illustré à la figure 6, le dégagement latéral 13 est supprimé et seul subsiste le logement cylindrique 11. De ce fait en fin de compression du connecteur 3 et de la tige 1 sur la surface d'appui 7, la surface légèrement déformée de la tige 1 ne s'appuie plus qu'en un point P4 sur la paroi cylindrique non interrompue du logement 11. Les deux points de contact P3 P4 de la tige 1 assurent encore un blocage satisfaisant de celle-ci, mais légèrement moins efficace que celui obtenu par les trois points P1, P2, P3.

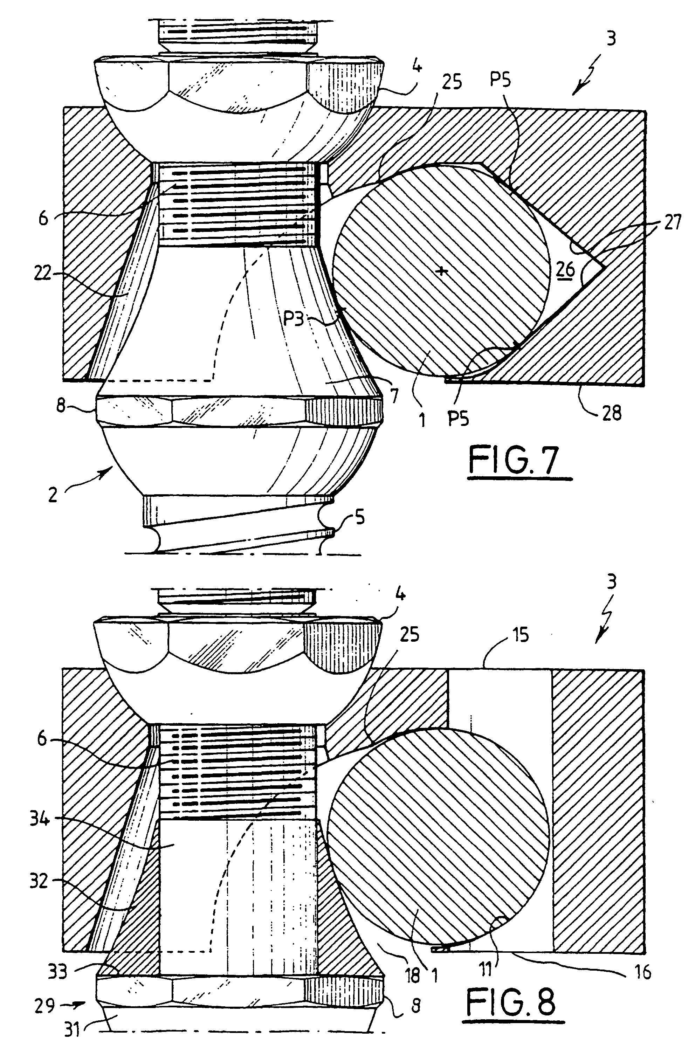

La troisième forme de réalisation de l'instrumentation, illustrée à la figure 7, diffère de la réalisation de la figure 1 par le fait que le logement cylindrique 11 est ici remplacé par un logement 26 ayant en section transversale un profil en V. Ainsi la tige 1 s'appuie sur chaque paroi 27 du logement 26 en deux points P5 qui avec le point de contact P3 sur la surface 7, assurent à la tige 1 un blocage efficace dans la position choisie, après serrage de l'écrou 4. La paroi inférieure 27 du logement 26 se termine par une pointe assurant avec l'arête 25, l'effet de clipsage de la tige 1.

Le quatrième mode de réalisation de l'instrumentation (figure 8) diffère des précédents en ce que l'organe d'ancrage 29 est constitué en deux parties 31, 32, dont l'une 32 porte la surface d'appui de la tige 1 et est montée de manière amovible sur l'autre partie 31. Plus précisément, la partie 32 est une bague à surface extérieure évasée, montée coulissante autour de la partie filetée 6 de l'organe d'ancrage 29, et pouvant venir en butée contre un épaulement annulaire transversal 33. Ce dernier est formé à la jonction d'une partie lisse 34 prolongeant la partie filetée 6 et de la forme de vissage 8. La surface de l'épaulement 33 est équipée de moyens antirotation pour la bague 32, par exemple des stries non représentées.

Par ailleurs, le mode d'assemblage et les résultats techniques obtenus par l'instrumentation de la figure 7 sont semblables aux précédents.

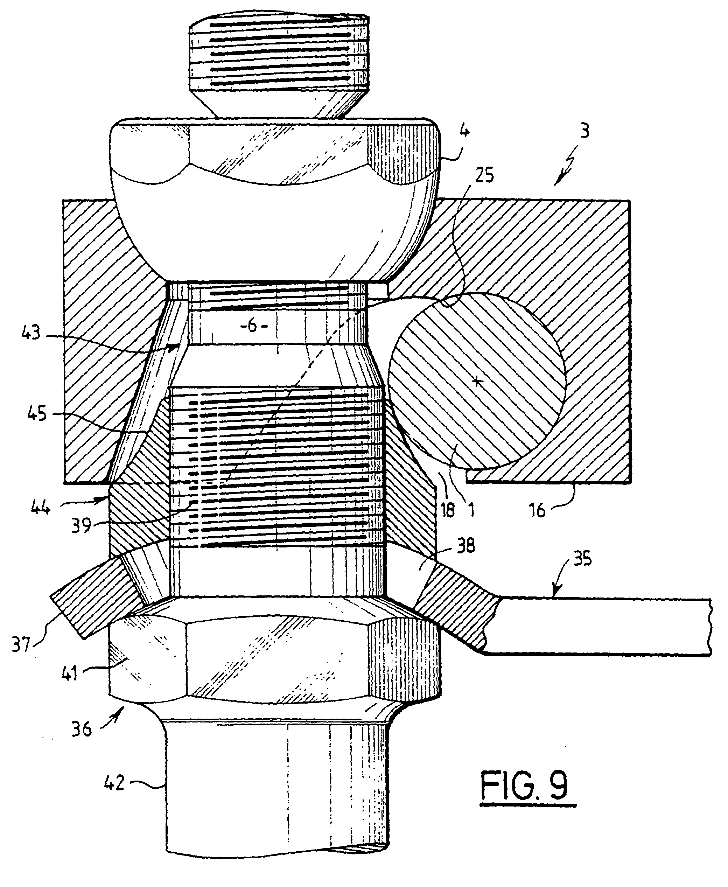

Dans le cinquième mode de réalisation de l'invention illustré à la figure 9, le dispositif comporte une pièce transversale supplémentaire 35 de liaison avec un autre élément (non représenté) de l'instrumentation, qui peut être un assemblage similaire d'un connecteur 3, d'une tige 1 et d'un organe d'ancrage 36. L'extrémité 37 de la pièce de liaison 36 forme une patte légèrement incurvée, percée d'un trou 38 de passage d'une partie filetée 39 de l'organe d'ancrage 36, et pouvant venir en appui sur la forme 41 de vissage osseux de l'organe d'ancrage 36.

La partie filetée 39 est raccordée à une zone lisse 43 elle-même prolongée par la partie filetée 6 de vissage de l'écrou de compression 4. Sur la partie filetée 39 peut venir se visser une bague-écrou 44 présentant une surface extérieure inclinée 45 d'appui et de blocage de la tige 1 dans le connecteur 3, cette surface 45 étant évasée dans l'exemple représenté. Ainsi l'extrémité 37 de la pièce de liaison 35 est interposée entre la bague-écrou 44 et la butée d'arrêt 41.

Outre les avantages techniques mentionnés précédemment, l'instrumentation selon l'invention présente celui d'être particulièrement compacte, grâce à l'appui direct de la tige 1 sur l'organe d'ancrage 2, et donc d'encombrement réduit par rapport aux instrumentations antérieures connues. Sa mise en oeuvre par le chirurgien est rendue aisée par l'ouverture d'introduction latérale 18 de la tige 1 et son clipsage dans le connecteur 3, assurant une sécurité accrue de manipulation des éléments de l'instrumentation.

L'invention est susceptible de diverses variantes d'exécution. Ainsi la surface d'appui inclinée (7, 45...) de la tige 1 peut être diversement réalisée, en étant constituée par exemple par un plat agencé sur une partie saillante de l'organe d'ancrage 2 ou bien présenter une forme conique, ou définie par une équation mathématique.

De même le débattement angulaire de l'organe d'ancrage 2 dans le connecteur 3, illustré à la figure 4, présente une amplitude qui peut varier en fonction de la différence entre les diamètres du trou évasé 22 et de l'ouverture cylindrique 24, et les dimensions correspondantes de la surface inclinée 7 et de la partie filetée 6. L'organe de compression 4 peut présenter une surface d'appui dans le connecteur 3 autre que sphérique, par exemple conique. Enfin la partie filetée 6 et la surface inclinée 7 peuvent être rapportées au reste de l'organe d'ancrage 2, par exemple par une extrémité filetée qui vient se visser à l'intérieur de la butée 8 et du début de la tige filetée 5.

## Revendications

1. Instrumentation d'ostéosynthèse rachidienne, comprenant au moins une tige vertébrale (1), des organes (2; 29, 36) d'ancrage vertébral ayant un axe longitudinal (XX) et échelonnés le long de la tige, un connecteur (3) de liaison entre la tige et chaque organe d'ancrage, ce dernier comportant une partie filetée (6) coopérant avec des moyens distincts de pression (4) pour bloquer ensemble la tige, les organes d'ancrage et les connecteurs, **caractérisée en ce que** chaque organe d'ancrage présente une surface d'appui (7) pour la tige, inclinée par rapport à l'axe longitudinal (XX) de l'organe d'ancrage de façon que la tige puisse prendre appui sur ladite surface entre deux points dont l'un situé du côté des moyens de pression (4) est plus proche de l'axe de l'organe d'ancrage que le second point, **en ce que** dans chaque connecteur est ménagé un logement (11) pour la tige, débouchant à ses deux extrémités à l'extérieur du connecteur et offrant au moins un point d'appui (P4) à la tige maintenue prisonnière par serrage des moyens de pression, entra ladite surface d'appui inclinée (7) et une paroi (12) du logement, **en ce que** chaque connecteur délimite une ouverture latérale (18) débouchant dans le logement (11) et permettant une introduction de la tige vertébrale transversalement ou radialement à sa direction longitudinale dans ce logement (11), et **en ce que** le logement (11) de la tige (1), la largeur de son ouverture latérale (18), et la tige (1) sont dimensionnés de telle sorte que , avant blocage du connecteur (3) sur la tige et sur l'organe d'ancrage, l'intervalle maximum (d5) entre l'organe d'ancrage (2) et le bord de l'ouverture d'introduction (18) de la tige reste toujours inférieur au diamètre (d4) de la tige, ce qui empêche un échappement de celle-ci hors du connecteur même après déclipsage.

2. Instrumentation selon la revendication 1, **caractérisée en ce que** la surface d'appui inclinée (7) est évasée, par exemple conique, ou constituée par un plat.

3. Instrumentation selon la revendication 2, **caractérisée en ce que** l'organe d'ancrage (29) est constitué en deux parties, dont l'une (32) porte la surface d'appui dé la tige (1) et est montée de manière amovible sur l'autre (31).

4. Instrumentation selon la revendication 3, **caractérisé en ce que** la partie de l'organe d'ancrage (29) portant la surface d'appui est une bague (32) à surface extérieure évasée, montée coulissante autour de l'organe d'ancrage et pouvant venir en butée contre un épaulement annulaire transversal (33) équipé de moyens anti-rotation, dudit organe d'ancrage.

5. Instrumentation selon la revendication 3, **caractérisée en ce que** la partie de l'organe d'ancrage portant la surface d'appui est une bague-écrou (44) vissée sur un filetage correspondant (39) de l'organe d'ancrage (36).

6. Instrumentation selon la revendication 5, **caractérisée en ce qu'**elle comporte une pièce transversale supplémentaire (35) de liaison avec un autre élément de l'instrumentation, dont une extrémité (37) est traversée par l'organe d'ancrage (36) et est interposée entre la bague-écrou (44) et une forme (41) de vissage de l'organe d'ancrage.

7. Instrumentation selon la revendication 1, **caractérisée en ce que** le logement (11) du connecteur (3) se prolonge, du côté opposé à l'ouverture d'introduction de la tige, par un dégagement central (13) délimitant deux griffes latérales (16) de part et d'autre dudit dégagement, les arêtes des parois de ces griffes et la surface d'appui (7) de l'organe d'ancrage (2, 29...) offrant trois points de contact (P1, P2, P3) à la tige après serrage des moyens (4) de pression et déformation de la tige sous l'action de ces derniers.

8. Instrumentation selon la revendication 7, **caractérisée en ce que** les griffes (16) enveloppent partiellement la tige (1) et leurs extrémités forment un bord de l'ouverture (18) d'introduction de la tige dans son logement (11), et **en ce que** ces extrémités sont reliées ensemble par un raccord (17) ou séparées de façon à constituer deux griffes distinctes.

9. Instrumentation selon la revendication 1, **caractérisée en ce que** la paroi (27) du logement (26) du connecteur (3) opposée à l'organe d'ancrage (2) possède un profil en V de façon à offrir à la tige (1) en section transversale, deux points d'appui (P5).

10. instrumentation selon l'une quelconque des revendications 1 à 8, **caractérisée en ce que** la distance (d3) entre les bords opposés de l'ouverture d'introduction (18) de la tigé (1) dans le connecteur (3) est sensiblement inférieure au diamètre (d4) de la tige, afin de créer un point dur (25) devant être franchi par la tige pour pénétrer dans son logement (11), et un effet de pince élastique de retenue de la tige par le connecteur.

11. Instrumentation selon la revendication 1, **caractérisée en ce que** dans le connecteur (3) est ménagé en vis-à-vis de la surface d'appui (7) de la tige (1) un trou évasé (22) pour le passage de l'organe d'ancrage (2; 29; 36), et ce trou évasé se prolonge par une ouverture (24) dont le diamètre est supérieur à celui d'une partie filetée (6) de l'organe d'ancrage, afin d'autoriser un débattement angulaire élevé de l'organe d'ancrage par rapport au connecteur.

12. Instrumentation selon la revendication 1, **caractérisée en ce que** lesdits moyens de pression (4) et de blocage possèdent une surface sphérique ou conique d'appui dans le connecteur, adaptée à une portée complémentaire (23) du connecteur.

13. Instrumentation selon la revendication 12, **caractérisée en ce que** les moyens de pression et de blocage sont formés par une vis pouvant se visser axialement dans l'organe d'ancrage (2; 29; 36).

## Patentansprüche

1. Vorrichtung zur Wirbelsäulenosteosynthese, umfassend wenigstens eine Wirbelsäulenstange (1), Wirbelverankerungselemente (2; 29, 36), die eine Längsachse (XX) aufweisen und entlang der Stange gestaffelt sind, und ein Verbindungselement (3) zur Verbindung zwischen der Stange und jedem Verankerungselement, wobei letzteres einen mit unterschiedlichen Druckmitteln (4) zusammenwirkenden Gewindeteil (6) umfasst, um zusammen die Stange, die Verankerungselemente und die Verbindungselemente festzuklemmen, **dadurch gekennzeichnet, dass** jedes Verankerungselement eine im Verhältnis zur Längsachse (XX) des Verankerungselementes so geneigte Stützfläche (7) für die Stange aufweist, dass die Stange sich auf der Fläche zwischen zwei Punkten aufstützen kann, von denen der eine, der sich an der Seite der Druckmittel (4) befindet, näher an der Achse des Verankerungselementes ist als der zweite Punkt, dass in jedem Verbindungselement eine Aufnahme (11) für die Stange ausgespart ist, welche an ihren beiden Enden zur Außenseite des Verbindungselements ausmündet und der durch Einspannung der Druckmittel festgehaltenen Stange wenigstens einen Stützpunkt (P4) zwischen der geneigten Stützfläche (7) und einer Wand (12) der Aufnahme bietet, dass jedes Verbindungselement eine laterale Öffnung (18) begrenzt, die in die Aufnahme (11) einmündet und ein zu seiner Längsrichtung transversales oder radiales Einführen der Wirbelsäulenstange in diese Aufnahme (11) erlaubt und dass die Aufnahme (11) der Stange (1), die Breite seiner lateralen Öffnung (18), und die Stange (1) derart bemessen sind, dass das maximale Intervall (d5) zwischen dem Verankerungselement (2) und dem Rand der Einführungsöffnung (18) der Stange vor dem Festklemmen des Verbindungselementes (3) auf der Stange und auf dem Verankerungselement immer kleiner als der Durchmesser (d4) der Stange bleibt, was ein Herausrutschen derselben aus dem Verbindungselement selbst nach dem Ausklinken verhindert.

2. Vorrichtung nach Anspruch 1, **dadurch gekennzeichnet, dass** die geneigte Stützfläche (7) aufgeweitet ist, zum Beispiel konisch, oder durch ein Flachprofil gebildet wird.

3. Vorrichtung nach Anspruch 2, **dadurch gekennzeichnet, dass** das Verankerungselement (29) aus zwei Teilen gebildet wird, von denen einer (32) die Stützfläche der Stange (1) trägt und abnehmbar auf dem anderen (31) angebracht ist.

4. Vorrichtung nach Anspruch 3, **dadurch gekennzeichnet, dass** der die Stützfläche tragende Teil des Verankerungselementes (29) ein gleitend um das Verankerungselement angebrachter Ring (32) mit aufgeweiteter Außenfläche ist, der gegen einen mit Antirotationsmitteln ausgerüsteten transversalen ringförmigen Absatz (33) des Elementes zum Anschlag kommen kann.

5. Vorrichtung nach Anspruch 3, **dadurch gekennzeichnet, dass** der die Stützfläche tragende Teil des Verankerungselementes eine auf ein entsprechendes Gewinde (39) des Verankerungselementes (36) aufgeschraubte Ringmutter (44) ist.

6. Vorrichtung nach Anspruch 5, **dadurch gekennzeichnet, dass** sie ein zusätzliches transversales Glied (35) zur Verbindung mit einem anderen Element der Vorrichtung umfasst, dessen eines Ende (37) von dem Verankerungselement (36) durchquert wird und zwischen die Ringmutter (44) und einen geformten Teil (41) zur Verschraubung des Verankerungselementes eingesetzt ist.

7. Vorrichtung nach Anspruch 1, **dadurch gekennzeichnet, dass** die Aufnahme (11) des Verbindungselementes (3) auf der der Einführungsöffnung der Stange gegenüber liegenden Seite durch eine zwei laterale Klauen (16) auf jeder Seite der Aussparung begrenzende zentrale Aussparung (13) verlängert ist, wobei die Kanten der Wände dieser Klauen und die Stützfläche (7) des Verankerungselementes (2, 29, ...) der Stange nach Einspannung der Druckmittel (4) und Deformation der Stange unter der Wirkung derselben drei Kontaktpunkte (P1, P2, P3) bieten.

8. Vorrichtung nach Anspruch 7, **dadurch gekennzeichnet, dass** die Klauen (16) die Stange (1) teilweise ummanteln und ihre Enden einen Rand der Einführungsöffnung (18) der Stange in ihre Aufnahme (11) bilden, und dass diese Enden miteinander durch ein Verbindungsstück (17) verbunden oder getrennt werden, so dass sie zwei unterschiedliche Klauen bilden.

9. Vorrichtung nach Anspruch 1, **dadurch gekennzeichnet, dass** die Wand (27) der Aufnahme (26) des Verbindungselementes (3) gegenüber dem Verankerungselement (2) ein V-Profil besitzt, so dass der Stange (1) im Querschnitt zwei Stützpunkte (P5) geboten werden.

10. Vorrichtung nach einem der Ansprüche 1 bis 8, **dadurch gekennzeichnet, dass** der Abstand (d3) zwischen den gegenüberliegenden Rändern der Einführungsöffnung (18) der Stange (1) in dem Verbindungselement (3) deutlich kleiner ist als der Durchmesser (d4) der Stange, um einen Hartpunkt (25), der von der Stange durchschritten werden muss, um in seine Aufnahme (11) einzudringen, und eine elastische Rückhalte-Zangenwirkung der Stange durch das Verbindungselement zu schaffen.

11. Vorrichtung nach Anspruch 1, **dadurch gekennzeichnet, dass** in dem Verbindungselement (3) gegenüber der Stützfläche (7) der Stange (1) ein aufgeweitetes Loch (22) für den Durchgang des Verankerungselementes (2; 29, 36) ausgespart ist und dieses aufgeweitete Loch durch eine Öffnung (24) verlängert ist, deren Durchmesser größer ist als der eines Gewindeteils (6) des Verankerungselementes, um ein erweitertes winkelförmiges Ausschwingen des Verankerungselementes relativ zu dem Verbindungselement zu erlauben.

12. Vorrichtung nach Anspruch 1, **dadurch gekennzeichnet, dass** die Druckmittel (4) und Mittel zum Festklemmen eine an eine komplementäre Auflagefläche (23) des Verbindungselements angepasste sphärische oder konische Stützfläche in dem Verbindungselement besitzen.

13. Vorrichtung nach Anspruch 12, **dadurch gekennzeichnet, dass** die Druckmittel und Mittel zum Festklemmen durch eine Schraube gebildet werden, die axial in das Verankerungselement (2; 29; 36) eingeschraubt werden kann.

## Claims

1. Spinal osteosynthesis instrumentation comprising at least one vertebral rod (1), vertebral anchor members (2; 29; 36) staggered along the rod and having a longitudinal axis (XX), and a connector (3) between the rod and each anchor member, the latter having a threaded portion (6) co-operating with separate pressure means (4) for locking together the rod, the anchor members and the connectors, **characterised in that** each anchor member has a bearing surface (7) for the rod inclined to the longitudinal axis (XX) of the anchor member so that the rod is able to bear on said surface between two points of which the point on the same side as the pressure means (4) is nearer the axis of the anchor member than the second point, **in that** each connector includes a housing (11) for the rod opening at both ends onto the exterior of the connector and offering at least one bearing point (P4) to the rod, which is trapped, on tightening the pressure means, between said inclined bearing surface (7) and a wall (12) of the housing, **in that** each connector delimits a lateral opening (18) leading into the housing (11) and allowing insertion of the vertebral rod into the housing (11) transversely or radially to its longitudinal direction, and **in that** the size of the housing (11) for the rod (1), the width of its lateral opening (18) and the size of the rod (1) are such that, before the connector (3) is locked onto the rod and onto the anchor member, the maximum gap (d5) between the anchor member (2) and the edge of the opening (18) for inserting the rod remains less than the diameter (d4) of the rod, which prevents the latter escaping from the connector, even after unclipping.

2. Instrumentation according to claim 1, **characterised in that** the inclined bearing surface (7) is flared, for example conical, or consists of a flat.

3. Instrumentation according to claim 2, **characterised in that** the anchor member (29) comprises two parts, of which one part (32) carries the bearing surface of the rod (1) and is removably mounted on the other part (31).

4. Instrumentation according to claim 3, **characterised in that** the part of the anchor member (29) carrying the bearing surface is a ring (32) with a flared exterior surface, slideably mounted around the anchor member and able to abut against a transverse annular shoulder (33) of said anchor member equipped with anti-rotation means.

5. Instrumentation according to claim 3, **characterised in that** the part of the anchor member carrying the bearing surface is a ring-nut (44) adapted to be screwed onto a corresponding thread (39) of the anchor member (36).

6. Instrumentation according to claim 5, **characterised in that** it includes an additional transverse part (35) for connecting it to another component of the instrumentation, one end (37) of which has the anchor member (36) passed through it and is disposed between the ring-nut (44) and a shaped portion (41) for screwing in the anchor member.

7. Instrumentation according to claim 1, **characterised in that** the housing (11) of the connector (3) is extended on the side opposite the opening for inserting the rod by a central recess (13) delimiting two lateral claws (16) on respective opposite sides of said recess, the edges of the walls of these claws and the bearing surface (7) of the anchor member (2, 29, ...) offering three points of contact (P1, P2, P3) to the rod after tightening of the pressure means (4) and deformation of the rod thereby.

8. Instrumentation according to claim 7, **characterised in that** the claws (16) partly envelop the rod (1) and their ends form an edge of the opening (18) for inserting the rod into its housing (11) and **in that** those ends are connected together by a connector (17) or separate, constituting two separate claws.

9. Instrumentation according to claim 1, **characterised in that** the wall (27) of the housing (26) of the connector (3) opposite the anchor member (2) has a V-shaped profile in cross-section in order to offer two bearing points (P5) to the rod (1).

10. Instrumentation according to any one of claims 1 to 8, **characterised in that** the distance (d3) between the opposite edges of the opening (18) for inserting the rod (1) into the connector (3) is significantly less than the diameter (d4) of the rod in order to create a hard spot (25) that the rod must overcome to enter its housing (11) and an elastic clamping effect whereby the rod is retained by the connector.

11. Instrumentation according to claim 1, **characterised in that** a flared hole (22) for the anchor member (2; 29; 36) to pass through is formed in the connector (3) facing the bearing surface (7) of the rod (1) and this flared hole is extended by an opening (24) whose diameter is greater than that of a threaded part (6) of the anchor member in order to allow considerable angular movement of the anchor member relative to the connector.

12. Instrumentation according to claim 1, **characterised in that** said pressure means (4) and said locking means have a spherical or conical surface adapted to bear on the connector and matching a complementary bearing surface (23) on the connector.

13. Instrumentation according to claim 12, **characterised in that** the pressure and locking means are formed by a screw adapted to be screwed axially into the anchor member (2; 29; 36).
